(19) Europäisches Patentamt
European Patent Office
Office européen des brevets

(11) Numéro de publication: **0 197 840**
**B1**

(12) # FASCICULE DE BREVET EUROPEEN

(45) Date de publication du fascicule du brevet: **01.08.90**

(51) Int. Cl.$^5$: **C 07 D 401/04**

(21) Numéro de dépôt: **86400639.0**

(22) Date de dépôt: **25.03.86**

(54) **(Imidazolyl-4) piperidines, leur préparation et leur application en thérapeutique.**

(30) Priorité: **26.03.85 FR 8504496**

(43) Date de publication de la demande:
**15.10.86 Bulletin 86/42**

(45) Mention de la délivrance du brevet:
**01.08.90 Bulletin 90/31**

(84) Etats contractants désignés:
**BE CH DE FR GB IT LI LU NL**

(56) Documents cités:
**US-A-4 217 350**

**CHEMICAL ABSTRACTS, vol. 80, no. 15, 15 avril 1974, page 389, abrégé 82801a, Columbus, Ohio, US; W. SCHUNACK: "Structure-action relationship of histamine analogs. 1. Histamine-like compounds with cyclized side chain" & ARCH. PHARM: (WEINHEIM, Ger.) 1973, 306(12), 934-42**

(73) Titulaire: **INSTITUT NATIONAL DE LA SANTE ET DE LA RECHERCHE MEDICALE (INSERM)**
**101, rue de Tolbiac**
**F-75654 Paris Cédex 13 (FR)**
(73) Titulaire: **UNIVERSITE DE CAEN**
**Esplanade de la Paix**
**F-14032 Caen (FR)**
(73) Titulaire: **SOCIETE CIVILE BIOPROJET**
**30, rue des Francs Bourgeois**
**F-75003 Paris (FR)**

(72) Inventeur: **Arrang, Jean-Michel Bat. 11-Res. du Chateau de Courcelle 160 av. Gal. Leclerc**
**F-91190 Gif/Yvette (FR)**
Inventeur: **Garbarg, Monique**
**10 rue Théodore de Banville**
**F-75017 Paris (FR)**
Inventeur: **Lancelot, Jean-Charles Maurice**
**Le Bourg**
**F-14400 Tour en Bessin (FR)**
Inventeur: **Lecomte, Jeanne-Marie**
**30 rue des Francs-Bourgeois**
**F-75003 Paris (FR)**
Inventeur: **Robba, Max-Fernand**
**12 rue du Père Sanson**
**F-14000 Caen (FR)**
Inventeur: **Schwartz, Jean-Charles**
**9 Villa Seurat**
**F-75014 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**EP 0 197 840 B1**

(74) Mandataire: **Moncheny, Michel et al**
**c/o Cabinet Lavoix 2 Place d'Estienne d'Orves**
**F-75441 Paris Cedex 09 (FR)**

**Description**

La présente invention concerne des (imidazolyl-4) pipéridines, leur préparation et leur application en thérapeutique, liés à un mécanisme d'action nouveau.

On connait d'après Arch. Pharm. 1973, 306(12), p. 934—42 (Chem. Abstr. *80*, n° 15, 82 801a) des Imidazolyl-4 pipéridines non substituées, ayant l'activité connue de l'histamine elle-même et qui sont utilisables comme intermédiaires de synthèse dans la présente invention. Par ailleurs, US—A—4 217 350 décrit des dérivés oxo-2 imidazolyl-pipéridines utilisables notamment comme antihypertenseurs.

Les composés de l'invention répondent à la formule générale

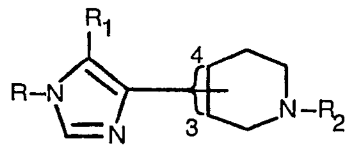

I

dans laquelle

$R_1$ représente un atome d'hydrogène ou un groupe méthyle ou éthyle,

R représente un atome d'hydrogène ou le radical $R_2$, et

$R_2$ représente un groupe alkyle droit ou ramifié de 1 à 6 atomes de carbone; un groupe (benzimidazolonyl-1)-3 propyle; un groupe de formule

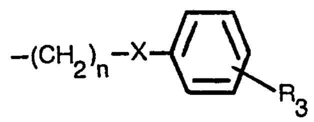

dans laquelle n est 0, 1, 2 ou 3, X est une liaison simple ou bien —O—, —S—, —NH—, —CO—, —CH=CH— ou

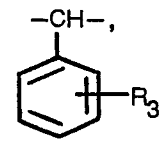

et

$R_3$ est H, $CH_3$, halogène, CN, $CF_3$ ou un groupe acyle $—COR_4$, $R_4$ étant un groupe alkyle droit ou ramifié de 1 à 6 atomes de carbone, cycloalkyle de 3 à 6 atomes de carbone ou phényle pouvant porter un substituant $CH_3$ ou F; ou bien un groupe de formule

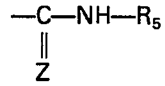

dans laquelle Z représente un atome O ou S ou un groupe divalent NH, $N—CH_3$ ou N—CN et $R_5$ représente un groupe alkyle droit ou ramifié de 1 à 8 atomes de carbone, cycloalkyle de 3 à 6 atomes de carbone pouvant porter un substituant phényle, cycloalkyl (3 à 6 C)-alkyle (1 à 3 C linéaire ou ramifié), un groupe phényle pouvant porter un substituant $CH_3$, halogène ou $CF_3$, un groupe phényl-alkyle (1 à 3 C linéaire ou ramifié), un groupe naphtyle, adamantyle ou p-toluènesulfonyle, ainsi que leurs sels pharmaceutiquement acceptables.

Un groupe de composés préférés selon l'invention est constitué par les composés de formule générale I dans laquelle $R_2$ est un groupe de formule

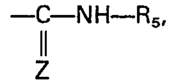

les symboles Z et $R_5$ ayant les significations données ci-dessus, et en particulier les composés dans lesquels Z est un atome O ou S ou un groupe NH.

Les composés de l'invention peuvent être préparés par réaction des (imidazolyl-4) pipéridines ou un de leurs dérivés alkylés de formule

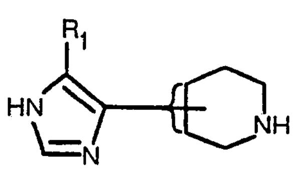

II

dans laquelle $R_1$ à la même signification que dans la formule I, soit avec un dérivé halogéné $R'_2X'$, $R'_2$ ayant la même signification de $R_2$ sauf le groupe de formule

$$-\underset{\underset{Z}{\|}}{C}-NH-R_5,$$

et X' désignant un halogène, soit, dans ce dernier cas, avec un dérivé de formule $Z=C=N-R_5$ dans laquelle Z et $R_5$ ont la même signification que dans la formule I, et le cas échéant, décomposition partielle du dérivé disubstitué (I; $R = R_2$) thermique ainsi préparé afin d'obtenir le dérivé monosubstitué correspondant (I; R = H).

Les composés de formule II sont connus et peuvent être préparés selon la méthode de Pyman (J. Chem. Soc. (1911) *99*, p. 668).

La réaction avec le dérivé halogéné $R'_2X'$ peut être effectuée de manière connue, par exemple par chauffage à des températures allant de 60 à 180°C, dans un solvant polaire tel que le diméthylformamide et en présence d'un accepteur d'acide tel qu'un carbonate alcalin ou alcalino-terreux.

La réaction de condensation avec le dérivé de formule $Z=C=N-R_5$ (en particulier isocyanate et isothiocyanate lorsque Z est O et S respectivement) peut être effectuée de manière connue, par exemple à des températures allant de 80 à 130°C, au reflux dans un solvant neutre tel que le toluène.

La décomposition thermique des composés disubstitués (I; $R = R_2$) peut être effectuée par chauffage et sublimation à des températures généralement supérieures à 200°C (par exemple 200 à 240°C) et sous pression réduite.

Les exemples suivants illustrent l'invention.

## Exemple 1

[(Cyclohexylaminocarbothioyl)-1 piperidinyl-4]-4 1H-imidazole

On porte au reflux pendant 2 heures 1,4 g (0,0092 mole) de (pipéridinyl-4)-4 1H-imidazole et 1,42 g (0,010 mole) d'isothiocyanate de cyclohexyle dans 100 cm3 de toluène anhydre. Le précipité formé après refroidissement est essoré, lavé à l'éther éthylique anhydre, séché et recristallisé dans le toluène. Poudre blanche. F(déc.) 170°C, p = 2 g. Rendement 74%.

Spectre IR (KBr): 3240 (NH). Bandes principales: 2920, 2840, 1520, 1440, 1350, 1330, 1250, 1175, 1090, 970, 830, 755 et 695 $cm^{-1}$.

Spectre de RMN: DMSO-$d_6$ δ H, imidazoles: 7,45 et 6,66 constante de couplage JH2—H5 = 0,90 Hz.

δ NH: 7,13 et 7,05 ppm.

δ H pipéridine et cyclohexyle: 4,61, 4,16, 2,98, 1,76 et 1,20 ppm.

Les exemples 6, 7, 31, 45, 46, 57, 59 à 62 du Tableau 1 sont préparés de façon analogue.

## Exemple 2

[(Cyclohexylamino-carbonyl)-1 piperidinyl-4]-4 cyclohexylamino-carbonayl-1 1H-imidazole

On porte au reflux pendant 1 heure 1 g (0,0066 mole) de (pipéridinyl-4)-4 1H-imidazole et 1,73 g (0,013 mole) d'isocyanate de cyclohexyle dans 80 cm3 de toluène anhydre. Après refroidissement de la solution, les cristaux formés sont essorés, lavés à l'éther éthylique anhydre, séchés et recristallisés dans l'acétonitrile. F: 192°C, p = 1,8 g.

Rendement: 67,9%.

Spectre IR (KBr): 3390 et 3210 (NH), 1695 et 1610 (C = 0).

Bandes principales: 3005, 2930, 2850, 1525, 1445, 1305, 1255, 1150, 1025, 970, 835 et 750 $cm^{-1}$.

Spectre de RMN: DMSO-$d_6$, δ H, imidazoles: 8.08 et 7,36 ppm.

δ NH: (doublets): 7,98 et 6,03 ppm.

δ H cyclohexyle et pipéridine: 4,00, 3,46, 2,68, 1,83 et 1,30 ppm.

Les exemples 12, 13, 14, 15, 16, 17, 18, 19, 20 et 21 du Tableau 1 sont synthétisés de façon analogue.

## Exemple 3

[(Cyclohexylamino-carbonyl)-1 piperidinyl-4]-4 1H-imidazole

0,6 g (0,0014 mole) de [(cyclohexylamino carbonyl-1) piperidinyl-4]-4 cyclohexylamino carbonyl-1 1H-imidazole obtenu selon l'exemple 2 sont chauffés à 220°C pendant 5 minutes puis sublimés sous 0,05 mmHg (6,5 Pa). Cristaux blancs. F = 166°C, p = 0,24 g. Rendement: 63%.

Spectre IR (KBr): 3200 (NH), 1610 (C = 0). Bandes principales à 2920, 2850, 1530, 1440, 1365, 1235, 1140, 1015, 975, 825, 765 et 630 $cm^{-1}$.

Spectre de RMN: DMSO-$d_6$: δ H imidazole: 7,36 et 6,60 ppm.

NH: 5,91 et 6,00 ppm.

H cyclohexyl et piperidine: 3,90, 3,83, 2,56, 1,66 et 1,10 ppm.

Les exemples 8 à 11, et 58 (Tableau 1) sont préparés de façon analogue.

Exemple 4

[(Cyclopropyl-carbonyl-4 phenyl)-1 piperidinyl-4]-4 1H-imidazole

3 g (0,019 mole) de (pipéridinyl-4)-4 1H-imidazole et 3,39 g (0,016 mole) de γ-chloro-p.fluorobutyrophénone sont agités dans 70 cm3 de diméthylformamide pendant 5 minutes, puis on ajoute 4 g (0,037 mole) de carbonate de sodium et 0,2 g (0,0012 mole) d'iodure de potassium et on porte la solution à 160°C pendant 3 heures. Après refroidissement, la solution est versée dans 200 cm3 d'eau et agité 1 heure. Le précipité formé est essoré, lavé à l'eau, séché et recristallisé dans l'acétonitrile. Poudre jaunâtre. F = 198°C, p = 0,6 g. Rendement: 11%.

Spectre IR (KBr): 3070 (NH), 1640 (C = 0), bandes principales à: 2920, 2820, 1595, 1385, 1230, 1100, 1035, 990, 830 et 765 $cm^{-1}$.

Spectre de RMN: DMSO-$d_6$ δ phényle: 7,86 et 6,95 ppm.

δ imidazole: 7,45 et 6,70 ppm.

δ (NH): 4,46 ppm. pipéridine: massif: 3,96, 3,33, 2,8 et 1,8 ppm.

δ cyclopropyle: 0,93 ppm.

Exemple 5

[(Propionyl-4 phenyl)-1 piperidinyl-4]-4 1H-imidazole

3 g (0,019 mole) de (pipéridinyl-4)-4-1H-imidazole et 2,88 g (0,019 mole) de para-fluoropropiophénone sont agités dans 70 cm3 de diméthylformamide pendant 5 minutes. On ajoute 4 g (0,037 mole) de carbonate disodique et 0,2 g (0,0012 mole) d'iodure de potassium et on porte la solution à 160°C pendant 3 heures. Après refroidissement, la solution est versée dans 200 cm3 d'eau et agitée pendant 1 heure. Le précipité formé est essoré, lavé à l'eau, séché et recristallisé dans l'acétonitrile.

Pourdre jaune, F = 210°C. Rendement: 13%.

Les exemples 22, 23, 24, 25 et 26 du Tableau 1 sont préparés de façon analogue.

Exemple 27

p-Fluorobenzyl-1 (imidazolyl-4)-4 piperidine

Une solution de 1,5 g d'(imidazolyl-4)-4 pipéridine dans 75 cm3 de diméthylformamide est additionnée de 2 g de carbonate de potassium et de 1,5 g de chlorure de p-fluorobenzyle. On chauffe ensuite à 80°C avec agitation pendant 2 heures. On filtre et évapore le filtrat sous pression réduite. Le résidu est recristallisé dans l'acétonitrile. Cristaux blancs, F = 232°C.

Rendement: 70%, $C_{15}H_{18}FN_3$ (M = 259,02).

Les exemples 28 à 30, 32 à 44 et 47 à 56 du Tableau 1 sont préparés de façon analogue.

Les composés des exemples précédents ainsi que d'autres composés de formule I donnés à titre d'exemples sont rassemblés dans le tableau 1 suivant.

La pipéridine est en position 4 par rapport à l'imidazole dans les exemples 1 à 58, et en position 3 dans les autres exemples (*).

Les composés ont été purifiés soit à l'aide du solvant indiqué soit comme suit:

(a) Purification par sublimation à 240°C sous 0,05 mm (6,5 Pa).

(b) Purification par sublimation à 210°C—220°C sous 0,05 mm.

(c) Purification par sublimation à 200°C sous 0,05 mm.

(d) Purification par sublimation à 200°C sous 0,05 mm.

TABLEAU 1

| Ex. N° | R | $R_1$ | $R_2$ | Pt. de fusion (°C) | Rdt. (%) | Solvant de cristallisation |
|---|---|---|---|---|---|---|
| 1 | H | H | 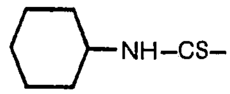 | 170 (déc.) | 74 | toluène |
| 2 | 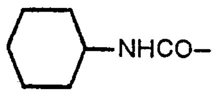 | | 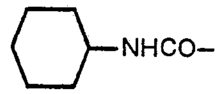 | 192 | 67,9 | acétonitrile |
| 3 | H | H | 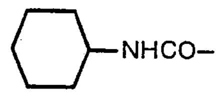 | 166 | 63 | (b) |
| 4 | H | H | 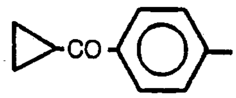 | 198 | 11 | acétonitrile |
| 5 | H | H | 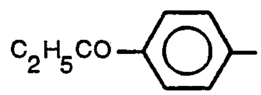 | 210 | 13 | acétonitrile |
| 6 | H | H | 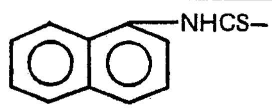 | 164 | 70 | acétonitrile |
| 7 | H | H | 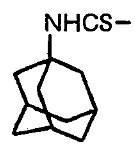 | 142 | 32 | toluène |
| 8 | H | H | 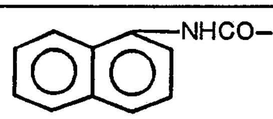 | 150 | 34 | (a) |
| 9 | H | H | 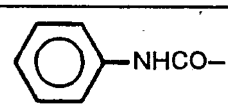 | 120 | 47 | (b) |
| 10 | H | H | 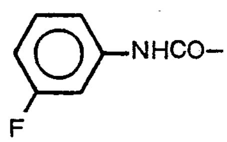 | 138 | 43 | (c) |
| 11 | H | H | 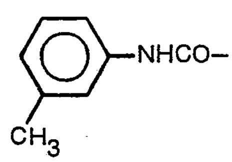 | 118 | 26 | (d) |
| 12 | $CH_3NHCO$— | H | $CH_3NHCO$— | 178 | 57 | toluène |
| 13 | 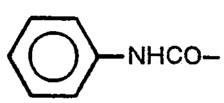 | H | 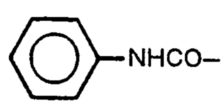 | 178 | 58 | toluène |

TABLEAU 1 (suite)

| Ex. N° | R | $R_1$ | $R_2$ | Pt. de fusion (°C) | Rdt. (%) | Solvant de cristallisation |
|---|---|---|---|---|---|---|
| 14 | 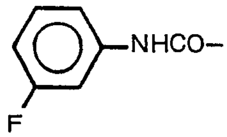 | H | 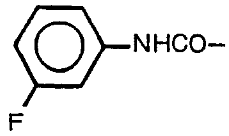 | 176 | 44 | toluène |
| 15 | 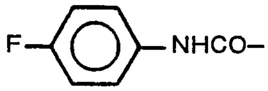 | H | 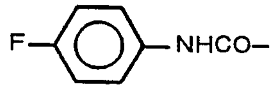 | 220 | 32 | toluène |
| 16 | 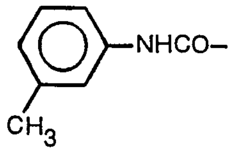 | H | 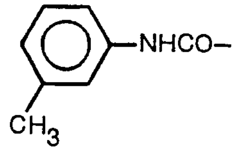 | 154 | 68 | acétonitrile |
| 17 | 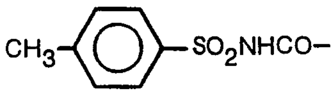 | H | 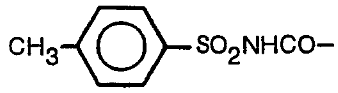 | 260 | 52 | toluène |
| 18 | 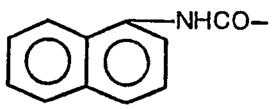 | H | 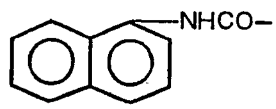 | 154 | 64 | toluène |
| 19 | 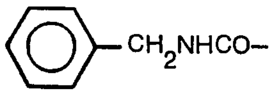 | H | 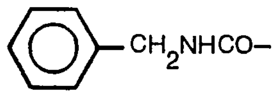 | 156 | 72 | toluène |
| 20 | 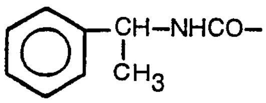 | H | 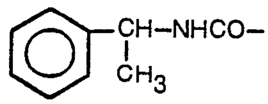 | 142 | 83 | toluène |
| 21 | 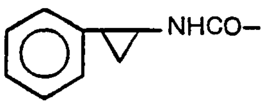 | H | 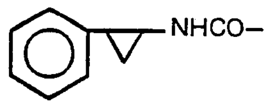 | 220 | 60 | toluène |
| 22 | 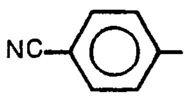 | H | 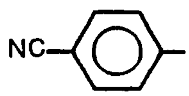 | 214 | 12 | toluène |
| 23 | 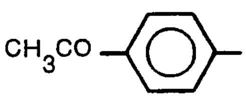 | H | 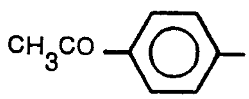 | 182 | 17 | acétone |
| 24 | 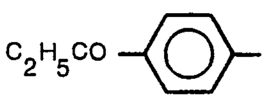 | H | 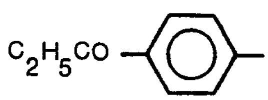 | 218 | 24 | acétonitrile |
| 25 | 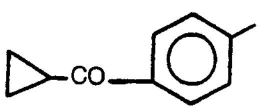 | H | 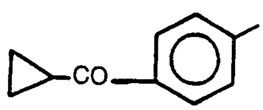 | 218 | 15 | acétonitrile |
| 26 | 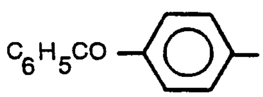 | H | 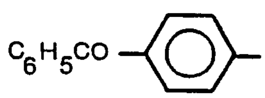 | 172 | 24 | acétone |

TABLEAU 1 (suite)

| Ex. N° | R | $R_1$ | $R_2$ | Pt. de fusion (°C) | Rdt. (%) | Solvant de cristallisation |
|---|---|---|---|---|---|---|
| 27 | H | H | 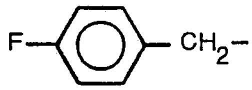 | 232 | 70 | acétonitrile |
| 28 | H | H | $-CH_3$ | 82 | 60 | éther éthylique |
| 29 | H | H | 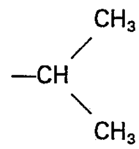 | 158 | 65 | acétate d'éthyle |
| 30 | H | $CH_3$ | 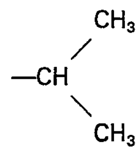 | 192 | 70 | acétonitrile |
| 31 | H | H | 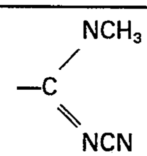 | 134 | 40 | acétate d'éthyle |
| 32 | H | H | 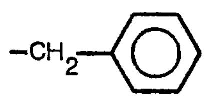 | 105 | 70 | éther éthylique |
| 33 | H | H | 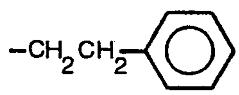 | 103 | 75 | éther éthylique |
| 34 | H | H | 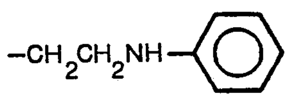 | 84 | 60 | éther éthylique |
| 35 | H | H | 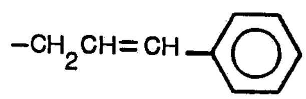 | 138 | 75 | acétate d'éthyle |
| 36 | H | $CH_3$ | 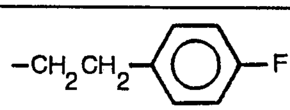 | 182 | 40 | acétonitrile |
| 37 | H | H | 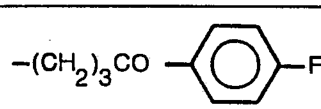 | 198 | 45 | éther éthylique |
| 38 | H | $CH_3$ | 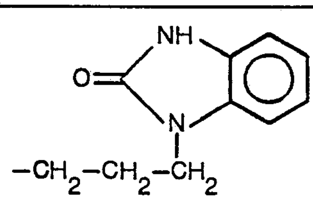 | 176 | 55 | acétonitrile |

TABLEAU 1 (suite)

| Ex. N° | R | $R_1$ | $R_2$ | Pt. de fusion (°C) | Rdt. (%) | Solvant de cristallisation |
|---|---|---|---|---|---|---|
| 39 | H | H | 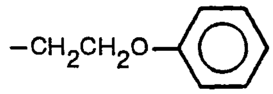 | 92 | 60 | éther éthylique |
| 40 | H | H | 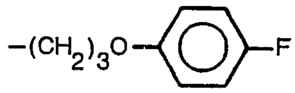 | 112 | 75 | acétate d'éthyle |
| 41 | H | H | $-CH_2CH_2CH(C_6H_5)_2$ | 96 | 70 | éther éthylique |
| 42 | H | H | $-(CH_2)_3CH(C_6H_4F\text{-}p)_2$ | 104 | 60 | éther éthylique |
| 43 | H | $CH_3$ | $CH_3$ | 186 | 70 | éther éthylique |
| 44 | H | H | 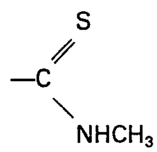 | 206 | 50 | acétate d'éthyle |
| 45 | H | $CH_3$ | 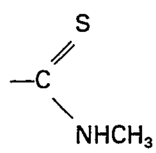 | 212 | 55 | acétate d'éthyle |
| 46 | H | $CH_3$ | 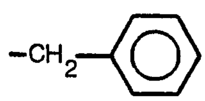 | 205 | 70 | éther éthylique |
| 47 | H | $CH_3$ | 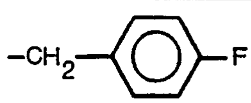 | 229 | 75 | éther éthylique |
| 48 | H | $CH_3$ | $-CH(C_6H_5)_2$ | 158 | 60 | acétone |
| 49 | H | $CH_3$ | $-(CH_2)_2CH(C_6H_5)_2$ | 195 | 70 | éther éthylique |
| 50 | H | H | 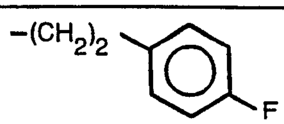 | 152 | 60 | acétonitrile |
| 51 | H | $CH_3$ | 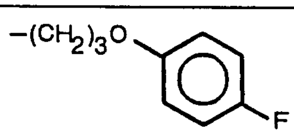 | 144 | 70 | acétate d'éthyle |
| 52 | H | $CH_3$ | 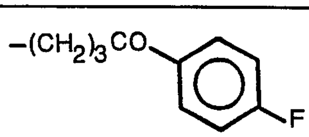 | 132 | 70 | éther éthylique |
| 53 | H | $CH_3$ | $-(CH_2)_3CH(C_6H_4p\text{-}F)_2$ | 132 | 75 | éther éthylique |

TABLEAU I (suite)

| Ex. N° | R | $R_1$ | $R_2$ | Pt. de fusion (°C) | Rdt. (%) | Solvant de cristallisation |
|---|---|---|---|---|---|---|
| 54 | H | H | 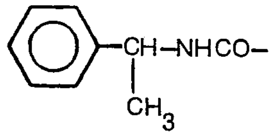 | 131 | 63 | toluène |
| 55 | H | H | 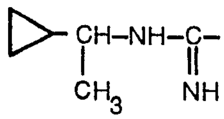 | 162 | 59 | toluène |
| 56* | H | H | 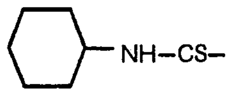 | 214 | 92 | acétonitrile |
| 57* | H | H | 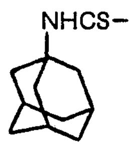 | 140 (déc.) | 65 | acétonitrile |
| 58* | H | H | 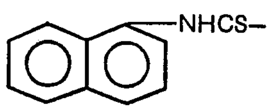 | 212 | 77 | acétonitrile |
| 59* | H | H | 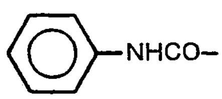 | 135 | 40 | acétonitrile |
| 60 | H | H | 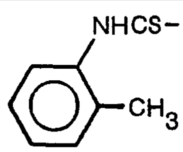 | 170 | 40 | acétonitrile |
| 61 | H | H | 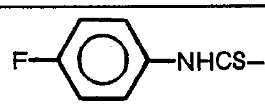 | 162 | 61 | acétonitrile |
| 62 | H | H | 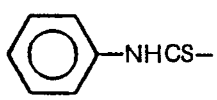 | 134 | 80 | toluène |
| 63 | H | H | 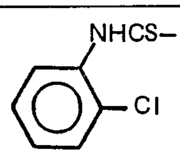 | 108 | 52 | toluène |
| 64 | H | H | 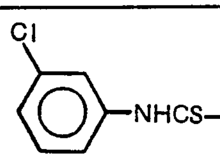 | 124 | 41 | toluène |
| 65 | H | H | 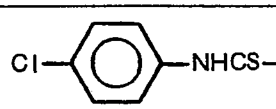 | 138 | 64 | toluène |

TABLEAU I (suite)

| Ex. N° | R | $R_1$ | $R_2$ | Pt. de fusion (°C) | Rdt. (%) | Solvant de cristallisation | |
|---|---|---|---|---|---|---|---|
| 66 | H | H | 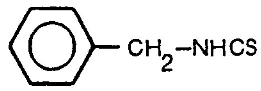 | 178 | 72 | acétonitrile | |
| 67 | 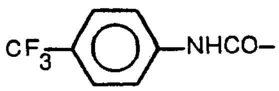 | H | 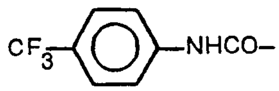 | 192 | 76 | acétonitrile | |
| 68 | H | H | 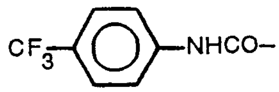 | 107 | 35 | sublimation 200°/0,05 mm | |
| 69 | H | H | 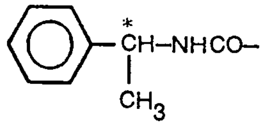 | 76 | 35 | Sublimation 200°/0,05 mm | (a) |
| 70 | H | H | 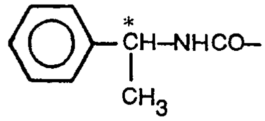 | 86 | 40 | Sublimation 210°/0,05 mm | (b) |
| 71 | H | H | 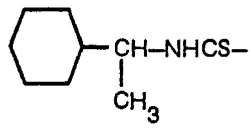 | 181 | 49 | toluène | [R.S] |
| 72 | H | H | 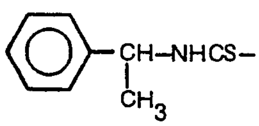 | 139 | 41 | toluène | [R.S] |
| 73 | H | H | 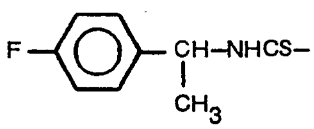 | 131 | 37 | acétonitrile | [R.S] |
| 74 | H | H | 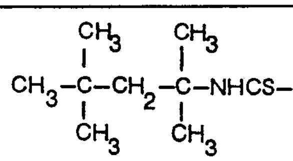 | 142 | 65 | acétonitrile | |

(a) isomère dextrogyre $\alpha_D = +35° 33$

(b) isomère lévogyre $\alpha_D = -36° 66$

Etude Pharmacologique

L'étude pharmacologique des composés de l'invention a permis de mettre en évidence pour la premiére fois, *in vitro* un blocage des récepteurs histaminergiques $H_3$ contrôlant la libération d'histamine cérébrale et, *in vivo*, une augmentation de la vitesse de renouvellement de l'histamine cérébrale, effets établissant notamment une action psychotrope.

Les épreuves pharmacologiques réalisées ont été les suivantes:

I — *Toxicite aigue*

La détermination de la mortalité chez la souris est observée à la suite de l'administration unique par voie intra-veineuse de doses croissantes de composés à tester.

La D.L.$_{50}$ pour tous les composés étudiés est supérieure à 100 mg/kg (i.p.). Par exemple, celle du dérivé N° 1 est de 100 mg/kg au bout d'une heure.

II — *Antagonisme de la stimulation par l'histamine des recepteurs $H_3$ controlant la liberation de l'histamine —$^3$H induite par depolarisation de coupes de cerveau de rat*

Le procédé décrit par Arrang et coll., (Nature 1983, 302: 838—837) est utilisé sur des coupes (0,3 mm d'épaisseur) de cortex cérébral de rats Wistar mâles (180—200 g) (IFFA-CREDO, France).

L'histamine exogène (à la concentration de 1 µM) produit une inhibition de libération d'environ 60%. Cet effet est progressivement reversé en présence des antagonistes $H_3$ ajoutés en concentrations croissantes. La concentration de ces derniers pour laquelle l'effet de l'histamine exogène est réduit de moitié ($CI_{50}$) est déterminée et la constante apparente d'inhibition (Ki) est alors calculée suivant Cheng et Prussof (Biochem. Pharmacol. 1973, *22*, 3099—3108) en tenant compte de la concentration efficace 50% de l'histamine ($CE_{50}$ = 0,13 µM). Les résultats sont rassemblés dans le Tableau 2 suivant. En outre, on a vérifié sur certains composés que l'inhibition est bien surmontable en accroissant la concentration d'histamine exogène.

TABLEAU 2

Constantes Apparentes de Dissociation (Ki) de Divers Derives Comme Antagonistes de l'Histamine sur les Autorecepteurs $H_3$ du Cerveau du Rat

| Exemples N° | Ki (nM) |
|---|---|
| 1 | 5 |
| 2 | 26 |
| 3 | 41 |
| 4 | 37 |
| 7 | 30 |
| 9 | 170 |
| 10 | 23 |
| 11 | 52 |
| 16 | 84 |
| 20 | 22 |
| 39 | 500 |
| 40 | 67 |
| 56 | 10 |
| 57 | 12 |
| 58 | 40 |

III — *Effets des antagonistes $H_3$ sur la vitesse de renouvellement de l'histamine cerebrale*

La vitesse de renouvellement de l'histamine dans le cortex cérébral de rat a été estimée par l'étude de la décroissance du taux de l'histamine après blocage de sa synthèse par l'α-fluorométhylhistidine (α-FMH), un inhibiteur irréversible de son enzyme de synthèse, l'histidine décarboxylase.

Des rats mâles de 80 à 100 g reçoivent par voie intrapéritonéale l'α-fluorométhylhistidine et le composé à tester, simultanément, chacun à la dose de 20 mg/kg. Une heure plus tard, les animaux sont sacrifiés et le cortex cérébral rapidement prélevé et homogénéisé dans 10 volumes de sucrose 0,32 M. L'homogénat ainsi obtenu est soumis à deux centrifugations successives, la première à $10^4$ g.mn et la deuxième à $2.10^5$ g.mn pour faire sédimenter la fraction enrichie en synaptosomes (Whittaker, Michaelson et Kirkland, Biochem. J., 1967, *90*, 293—305). Après resuspension et traitement par ultrasons de cette fraction dans 10 volumes de tampon phosphate ($K_2/K$) 20 mM, pH 7,8, une partie aliquote est congelée pour dosage ultérieur de l'activité de l'histidine décarboxylase (Baudry, Martres et Schwartz, J. Neurochem., *21*, 1301—1309, 1973), une autre partie est chauffée 10 minutes à 95°C puis centrifugée à $10^4$ g × mn. L'histamine endogène est dosée dans le surnageant par un dosage radioenzymatique qui permet de transformer l'histamine en N-télé-méthylhistamine tritiée grâce à une enzyme purifiée à partir de reins de rat, l'histamine -N-méthyltransférase et à un donneur de radical méthyle tritié, la S-adénosyl-méthionine. La N-télé-méthylhistamine ainsi formée est sélectivement extraite en mileu alcalin, dans du chloroforme avant mesure par scintillation liquide du produit tritié (Taylor et Snyder, J. Pharmacol. Exp. Ther., *137*, 619—633, 1971). La vitesse de renouvellement de l'histamine est calculée à partir de la différence entre ses taux avant et après administration d'α-fluorométhylhistidine.

Chez les témoins celles-ci correspond à 15 ng/g/h contre environ 30 ng/g/h après traitement par un antagoniste des récepteurs $H_3$ (Tableau 3).

De plus le dérivé de l'exemple N°1 administré seul à la dose de 20 mg/kg induit une baisse significative du taux d'histamine de la fraction synaptosomale.

TABLEAU 3

Effets des Exemples N$^{os}$ 1 et 3 sur la Vitesse de Renouvellement de l'Histamine Dans la Fraction Synaptosomale ($P_2$) de Cortex Cerebral de Rat

| Traitement | Histamine | Depletion par α-FMH | |
|---|---|---|---|
| Véhicule | 51,6 ± 3,0 | 0 | |
| α-FMH (20 mg/kg) | 37,1 ± 2,7 | 14,5 ± 4,0 | |
| Exemple N°1 (20 mg/kg) | 39,3 ± 2,1** | 12,3 ± 3,7 | |
| α-FMH (20 mg/kg) + Dérivé N°1 (2 mg/kg) | 23,2 ± 2,7* | 28,4 ± 4,0 | (+96%) |
| + Dérivé N°1 (10 mg/kg) | 21,9 ± 1,0* | 29,7 ± 3,2*** | (+105%) |
| + Dérivé N°1 (20 mg/kg) | 26,4 ± 1,4* | 25,2 ± 3,3 | (+74%) |
| α-FMH (20 mg/kg) + Exemple N° 3 (20 mg/kg) | 24,9 ± 1,2* | 26,7 ± 3,2*** | (+84%) |

* P < 0,01 par rapport aux animaux traités par -FMH seul.
** P < 0,03 avec les animaux sans traitement.
*** P < 0,05 par rapport aux animaux traités par -FMH chaque groupe comprend 7—20 rats.

Les résultats de ces études mettent en évidence la faible toxicité, les intéressantes propriétés d'antagonistes des récepteurs $H_3$ de l'histamine et la faculté d'augmenter la vitesse de renouvellement de l'histamine cérébrale des dérivés de l'invention, propriétés qui en font les premiers composés de ce type utiles en mèdecine humaine et vétérinaire. Leurs applications thérapeutiques concernent notamment le système nerveux central et les organes périphériques dont les fonctions sont régulées par les récepteurs $H_3$ de l'histamine.

Le médicament de l'invention est administrable à l'homme par voie orale, perlinguale, nasale, rectale et parentérale, le principe actif étant associé à un excipient ou véhicule thérapeutique convenable.

Chaque dose unitaire contient avantageusement de 0,5 à 100 mg de principe actif, les doses administrables journellement pouvant varier de 0,5 à 200 mg de principe actif.

**Revendications**

1. Composés répondant à la formule générale

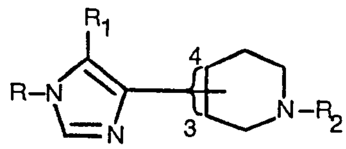

I

dans laquelle

$R_1$ représente un atome d'hydrogène ou un groupe méthyle ou éthyle,

R représente un atome d'hydrogène ou le radical $R_2$, et

$R_2$ représente un groupe alkyle droit ou ramifié de 1 à 6 atomes de carbone; un groupe (benzimidazolonyl-1)-3 propyle; un groupe de formule

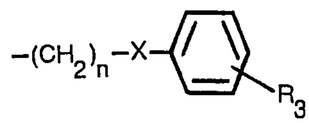

dans laquelle n est 0, 1, 2 ou 3, X est une liaison simple ou bien —O—, —S—, —NH—, —CO—, —CH=CH— ou

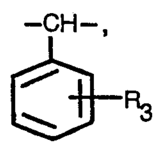

et

$R_3$ est H, $CH_3$, halogène, CN, $CF_3$ ou un groupe acyle —$COR_4$, $R_4$ étant un groupe alkyle droit ou ramifié de 1 à 6 atomes de carbone, cycloalkyle de 3 à 6 atomes de carbone ou phényle pouvant porter un substituant $CH_3$ ou F; ou bien un groupe de formule

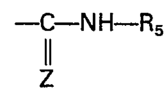

dans laquelle Z représente un atome O ou S ou un groupe divalent NH, N—$CH_3$ ou N—CN et $R_5$ représente un groupe alkyle droit ou ramifié de 1 à 8 atomes de carbone, cycloalkyle de 3 à 6 atomes de carbone pouvant porter un substituant phényle, cycloalkyl (3 à 6 C)-alkyle (1 à 3 C linéaire ou ramifié), un groupe phényle pouvant porter un substituant $CH_3$, halogène ou $CF_3$, un groupe phényl-alkyle (1 à 3 C linéaire ou ramifié), un groupe naphtyle, adamantyle ou p-toluènesulfonyle, ainsi que leurs sels pharmaceutiquement acceptables.

2. Composés selon la revendication 1, dans laquelle $R_2$ représente un groupe de formule

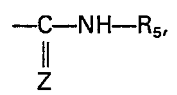

ayant la même signification que dans la formule I.

3. Composés selon la revendication 2, dans laquelle Z est O ou S ou un groupe NH.

4. Le (cyclohexylaminocarbothioyl-1 pipéridinyl-4)-4 1H-imidazole.

5. Le (cyclohexylaminocarbonyl-1 pipéridinyl-4)-4 cyclohexylaminocarbonyl-1 1H-imidazole.

6. Le (cyclohexylaminocarbonyl-1 pipéridinyl-4)-4 1H-imidazole.

7. Le [(cyclopropylcarbonyl-4 phényl)-1 pipéridinyl-4]-4 1H-imidazole.

8. L'[(adamantyl-1 aminocarbothioyl)-1 pipéridinyl-4]-4 1H-imidazole.

9. Le (m-fluorophénylaminocarbonyl-1 pipéridinyl-4)-4 1H-imidazole.

10. Le (α-méthylbenzylaminocarbonyl-1 pipéridinyl-4)-4 α-méthylbenzylaminocarbonyl-1 1H-imidazole.

11. Procédé de préparation des composés selon la revendication 1, caractérisé en ce que l'on fait réagir l'(imidazolyl-4)-4 pipéridine ou un de ses dérivés alkylés de formule

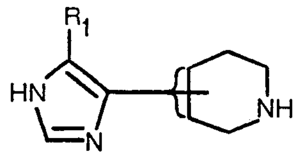

II

dans laquelle $R_1$ a la même signification que dans la formule I, soit avec un dérivé halogéné $R'_2X'$, $R'_2$ ayant la même signification que $R_2$ sauf le groupe de formule

$$-\underset{\underset{Z}{\|}}{C}-NH-R_5,$$

et X' désignant un halogène, soit, dans ce dernier cas, avec un dérivé de formule $Z{=}C{=}N{-}R_5$ dans laquelle Z et $R_5$ ont la même signification que dans la formule I, et le cas échéant, on effectue une décomposition partielle du dérivé disubstitué (I; $R = R_2$) ainsi préparé afin d'obtenir le dérivé monosubstitué correspondant (I; R = H).

12. Composition pharmaceutique contenant un composé selon l'une quelconque des revendications 1 à 10 et un excipient ou véhicule thérapeutiquement compatible.

**Patentansprüche**

1. Verbindungen der allgemeinen Formel

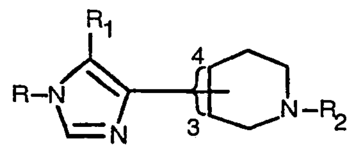

I

in der

$R_1$ ein Wasserstoffatom oder eine Methyl- oder Ethylgruppe,

R ein Wasserstoffatom oder eine Gruppe $R_2$ und

$R_2$ eine geradkettige oder verzweigte Alkylgruppe mit 1 bis 6 Kohlenstoffatomen, eine 3-(1-Benzimidazolonyl)-propyl-Gruppe; eine Gruppe der Formel

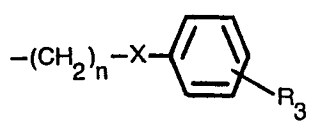

in der n 0, 1, 2 oder 3, X eine Einfachbindung oder eine Gruppe der Formeln —O—, —S—, —NH—, —CO—, —CH=CH— oder

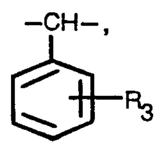

und

$R_3$ ein Wasserstoffatom, eine Methylgruppe, ein Halogenatom, eine CN-Gruppe, eine $CF_3$-Gruppe oder eine Acyl-$COR_4$-Gruppe, worin $R_4$ für eine geradkettige oder verzweigte Alkylgruppe mit 1 bis 6 Kohlenstoffatomen, eine Cycloalkylgruppe mit 3 bis 6 Kohlenstoffatomen oder eine Phenylgruppe, die einen $CH_3$- oder F-Substituenten tragen kann, steht, darstellen; oder eine Gruppe der Formel

$$-\underset{\underset{Z}{\|}}{C}-NH-R_5,$$

in der Z ein Sauerstoffatom oder ein Schwefelatom oder eine zweiwertige Gruppe der Formeln NH, $N{-}CH_3$ oder N—CN und $R_5$ eine geradkettige oder verzweigte Alkylgruppe mit 1 bis 8 Kohlenstoffatomen, eine Cycloalkylgruppe mit 3 bis 6 Kohlenstoffatomen, die einen Phenyl-Substituenten aufweisen kann, eine Cycloalkyl-alkylgruppe (mit 3 bis 6 Kohlenstoffatomen im Cycloalkylrest und einer geradkettigen oder

verzweigten Alkylgruppe mit 1 bis 3 Kohlenstoffatomen), eine Phenylgruppe, die eine Methylgruppe, ein Halogenatom oder eine $CF_3$-Gruppe als Substituenten aufweisen kann, eine Phenyl-alkyl-Gruppe (mit einer geradkettigen oder verzweigten Alkylgruppe mit 1 bis 3 Kohlenstoffatomen), eine Naphthylgruppe, eine Adamantylgruppe oder p-Toluolsulfonylgruppe darstellen, bedeuten, sowie deren pharmazeutisch annehmbare Salze.

2. Verbindungen nach Anspruch 1, worin $R_2$ eine Gruppe der Formel

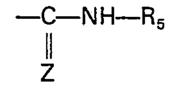

darstellt, welche die bezüglich der allgemeinen Formel I angegebenen Bedeutungen besitzt.

3. Verbindungen nach Anspruch 2, worin Z ein Sauerstoffatom, ein Schwefelatom oder eine NH-Gruppe bedeutet.

4. 4-(1-Cyclohexylaminocarbothioyl-4-piperidinyl)-1H-imidazol.

5. 4-(1-Cyclohexylaminocarbonyl-4-piperidinyl)-1-cyclohexylaminocarbonyl-1H-imidazol.

6. 4-(1-Cyclohexylaminocarbonyl-4-piperidinyl)-1H-imidazol.

7. 4-[1-(Cyclopropylcarbonyl-phenyl)-4-piperidinyl]-1H-imidazol.

8. 4-[1-(1-Adamantyl-aminocarbothioyl)-4-piperidinyl]-1H-imidazol.

9. 4-(1-m-Fluorphenylaminocarbonyl-4-piperidinyl)-1H-imidazol.

10. 4α-(1α-Methylbenzylaminocarbonyl-4-piperidinyl)-1-methylbenzylaminocarbonyl-1H-imidazol.

11. Verfahren zur Herstellung der Verbindungen nach Anspruch 1, dadurch gekennzeichnet, daß man 4-(4-Imidazolyl)-piperidin oder eines seiner alkylierten Derivate der Formel II

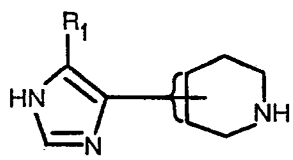

II

in der $R_1$ die bezüglich der allgemeinen Formel I angegebenen Bedeutungen besitzt, entweder mit einem Halogenderivat der Formel $R'_2X'$, worin $R'_2$ die gleichen Bedeutungen besitzt wie die Gruppe $R_2$ mit Ausnahme der Gruppe der Formel

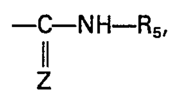

und X' ein Halogenatom darstellt, oder im letzteren Fall mit einem Derivat der Formel $Z{=}C{=}N{-}R_5$, in der Z und $R_5$ die bezüglich der Formel I angegebenen Bedeutungen besitzen, umsetzt und gewünschtenfalls eine teilweise Zersetzung des in dieser Weise hergestellten disubstituierten Derivats (I; $R = R_2$) bewirkt, um das entsprechende monosubstituierte Derivat (I; $R = H$) zu bilden.

12. Pharmazeutische Zubereitung enthaltend eine Verbindung nach einem der Ansprüche 1 bis 10 und ein therapeutisch verträgliches Bindemittel oder Trägermaterial.

**Claims**

1. Compounds corresponding to the general formula

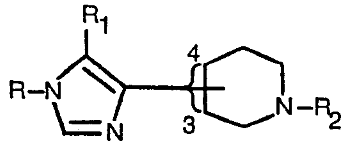

in which

$R_1$ denotes a hydrogen atom or ethyl group,

R denotes a hydrogen atom or the radical $R_2$, and

$R_2$ denotes a linear or branched alkyl group having 1 to 6 carbon atoms; a 3-1(1-benzimidazolonyl)propyl group; a group of the formula

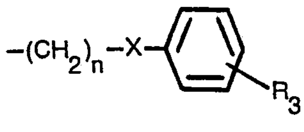

I

in which n is 0, 1, 2 or 3, x is a single bond or alternatively —O—, —S—, —NH—, —CO—, —CH=CH— or

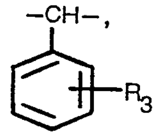

and

$R_3$ is H, $CH_3$, halogen, CN, $CF_3$ or an acyl group —$COR_4$ $R_4$ being a linear or branched alkyl group having 1 to 6 carbon atoms, a cycloalkyl group having 3 to 6 carbon atoms or a phenyl group which may have a $CH_3$ or F substituent; or alternatively a group of the formula

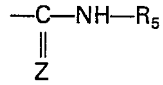

in which Z denotes an O or S atom or a divalent group NH, N—$CH_3$ or N—CN and $R_5$ denotes a linear or branched alkyl group having 1 to 8 carbon atoms, a cycloalkyl group having 3 to 6 carbon atoms which may have a phenyl substituent, a cycloalkyl group (3 to 6 C), an alkyl group (1 to 3 C, linear or branched), a phenyl group which may a $CH_3$, halogen or $CF_3$ substituent, a phenylalkyl group (1 to 3 C, linear or branched) a naphthyl, adamantyl or p-toluenesulphonyl group, as well as the pharmaceutically acceptable salts thereof.

2. Compounds according to Claim 1, in which $R_2$ denotes a group of formula

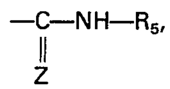

having the same meaning as in the formula I.

3. Compounds according to Claim 2, in which Z is O or S or an NH group.

4. 4-(1-cyclohexylaminothiocarbonyl-4-piperidyl)-1H-imidazole.

5. 4-(1-cyclohexylaminocarbonyl-4-piperidyl)-1-cyclohexylaminocarbonyl-1H-imidazole.

6. 4-(1-cyclohexylaminocarbonyl-4-piperidyl)-1H-imidazole.

7. 4-[1-(4-cyclopropylcarbonylphenyl)-4-piperidyl]-1H-imidazole.

8. 4-[1-(1-adamantylaminothiocarbonyl)-4-piperidyl]-1H-imidazole.

9. 4-[1-(m-fluorophenylaminocarbonyl)-4-piperidyl]-1H-imidazole.

10. 4-[1-(α-methylbenzylaminocarbonyl)-4-piperidyl]-1-(α-methylbenzylaminocarbonyl)-1H-imidazole.

11. Process for preparing the compounds according to Claim 1, characterised in that 4-(4-imidazolyl)piperidine or an alkyl derivative thereof of formula

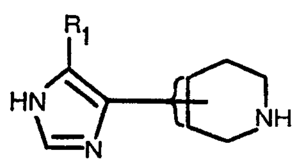

II

in which $R^1$ has the same meaning as in the formula 1, is reacted either with a halogenated derivative $R'_2X'$, $R'_2$ having the same meaning as $R_2$ except for the group of formula

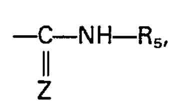

and X' denoting a halogen, or, in this latter case, with a derivative of formula Z=C=N—$R_5$ in which Z and $R_5$ have the same meaning as in the formula 1, and, where appropriate, the disubstituted derivative (I; R = $R_2$) thereby prepared is partially decomposed to obtain the corresponding monosubstituted derivative (I; R = H).

12. Pharmaceutical composition containing a compound according to any one of Claims 1 to 10 and a therapeutically compatible excipient or vehicle.